Europäisches Patentamt

European Patent Office

Office européen des brevets

(11) Publication number: **0 382 576**
**A1**

# EUROPEAN PATENT APPLICATION

(21) Application number: **90301461.1**

(51) Int. Cl.5: **C08B 15/02, C08B 11/20**

(22) Date of filing: **12.02.90**

(30) Priority: **10.02.89 US 309387**
**23.06.89 US 370629**

(43) Date of publication of application:
**16.08.90 Bulletin 90/33**

(84) Designated Contracting States:
**AT BE CH DE DK ES FR GB GR IT LI LU NL SE**

(71) Applicant: **Alko Ltd.**
**P.O. Box 350**
**SF-00101 Helsinki(FI)**

(72) Inventor: **Timonen, Maritta**
**Alko Ltd., P.O.B. 350**
**SF-00101 Helsinki(FI)**
Inventor: **Turunen, Marja**
**Alko Ltd., P.O.B. 350**
**SF-00101 Helsinki(FI)**
Inventor: **Vaara, Martti**
**Alko Ltd., P.O.B. 350**
**SF-00101(FI)**
Inventor: **Vaara, Timo**
**Alko Ltd., P.O.B. 350**
**SF-00101 Helsinki(FI)**

(74) Representative: **Collier, Jeremy Austin Grey et al**
**J.A.Kemp & Co. 14, South Square Gray's Inn**
**London WC1R 5EU(GB)**

(54) Cellulose derivative hydrolysate and fractions thereof.

(57) A water soluble degradation product of a cellulose derivative and fractions thereof comprising a cellulose derivative degraded to form a mixture of oligomers having an average degree of polymerization in the range of 3 to 300 and a molecular weight of 500 to 100000 and a method for producing and using such degradation products and fractions thereof.

EP 0 382 576 A1

## CELLULOSE DERIVATIVE HYDROLYSATE AND FRACTIONS THEREOF

This invention relates to cellulose derivative hydrolysates and fractions thereof. The invention also provides methods for producing the same, as well as novel uses for the hydrolysate and its fractions, which can be prepared by any of enzymatic, chemical or physical treatments of cellulose derivatives.

## BACKGROUND OF THE INVENTION

Cellulose derivatives such as carboxymethylcellulose, methylcellulose, methylethylcellulose, hydroxypropylmethylcellulose and hydroxypropylcellulose are non-caloric (non-metabolizable by humans or intestinal flora in human beings), odorless, tasteless water-soluble or water suspendable polymers derived from cellulose. These cellulose derivatives may act as thickeners, binders, stabilizers, suspending agents or flow control agents. They form films resistant to oils, greases and organic solvents. They dissolve rapidly in cold and hot water and are physiologically inert. These functions make them suitable for use in a broad range of applications in food, pharmaceutical, cosmetic, paper and other industries.

For such applications, degradation of cellulose derivatives is normally considered undesirable and to be avoided. Cellulolytic and viscosity reducing treatments on cellulose derivatives have been deliberately avoided in the past.

Enzymatic hydrolysis of cellulose derivatives have been studied in the past in the context of synergism studies among combinations of enzymes, the possible indexing of substituent distribution patterns, the effect of various substituents on enzymatic hydrolysis and the like. Such studies have been published in the following: Chouchon et. al., Biotech. Bioeng., Vol 26, pp. 988-991 (1984); Henrissat et. al., Biotechnology, Vol. 3, pp. 722-726 (1985); Chetkarov et. al., Monatshefte Fur Chemie, Vol. 116, pp. 1433-45 (1985); Chetkarov et. al., Monatshefte Fur Chemie, Vol. 117, pp. 1021-1026 (1986); Wirick, J. Polym. Sci., Part A-1, Vol. 6, pp. 1195-1974 (1968); Bhattacharjee, J. Polym. Sci., Part C, Vol. 36, pp. 509-521 (1971). Reduction of chain length determinations have also been studied. Almin et. al., Arch. Biochem. Biophys., pp. 124, 129 (1968); Ghose, Biotech. Bioeng., Vol. 11, pp. 239 (1969).

The present invention deals with a novel degradation of cellulose derivatives and products incorporating such degraded products. We have found that some of the properties of these cellulose derivatives can be further improved by degradation into low molecular weight polymers or oligomeric mixtures. These oligomeric mixtures and fractions (of the total mixture of oligomers into further separated mixtures of oligomers of varying chain length) thereof are more advantageous for several different applications in food, pharmaceutical, paper, cosmetic and textile industries than high molecular weight cellulose derivatives or their unhydrolysed counterparts.

## SUMMARY OF THE INVENTION

The invention discloses a novel water soluble mixture of oligomers derived from cellulose derivatives as well as fractions of the mixtures of oligomers or suspendable low molecular weight polymers obtained from the initial degradative process.

The oligomeric mixtures can be made from several different cellulose derivatives, the most preferred raw material being carboxymethylcellulose. The oligomeric mixture can be prepared by different modified and unmodified cellulolytic enzymes, the most preferred sources of the enzyme being strains of Trichoderma reesei, Aspergillus and Penicillium.

The oligomeric mixtures and fractions thereof have several applications in food, paper, pharmaceutical and cosmetic industry, but they are especially useful as calorie saving or low caloric substitutes in a wide range of food stuffs.

In accordance with the invention there is provided a water soluble mixture of oligomers derived from a cellulose derivative comprising a cellulose derivative degraded to form a mixture of oligomers having an average degree of polymerization in the range of 3 to 300 and a molecular weight of 500 to 100000. The soluble cellulose derivative is preferably selected from the group of carboxymethylcellulose, methylcellulose, methylethylcellulose, hydroxypropylmethylcellulose and hydroxypropylcellulose and mixtures thereof. The cellulose derivative may be degraded by enzymatic, chemical or physical agents/mechanisms. In embodiments where an enzyme preparation is utilized, the enzyme preparation is typically selected from the group of cellulases, modified cellulases and mixtures thereof.

In embodiments where degradation of a cellulose derivative is to be effected by chemical or physical means, chemical hydrolysis, chemical oxidation and physical depolymerization are preferred mechanisms for achieving the desired oligomeric mixtures according to the invention.

An enzyme preparation may be a cellulase or modified cellulase (i.e., modified to remove or prevent the formation of mono- and disaccharides producing enzymes in the cellulase preparation in the first instance, e.g., by genetic alteration of the microorganism from which the cellulase preparation is prepared) preferably produced from microorganisms selected from the group of Trichoderma, Aspergillus and Penicillium. Most preferably a cellulase preparation is derived from Trichoderma reesei from which at least one of beta-glucosidase and cellobiohydrolase activities have been removed. An enzyme preparation most preferably comprises endo- 1, 4- beta-glucanase.

The invention also provides a method for producing a mixture of oligomers from cellulose derivatives comprising the steps of: selecting a cellulose derivative; selecting a cellulolytic agent which degrades the selected cellulose derivative into a mixture of oligomers having an average degree of polymerization in the range of 3 to 300 and a molecular weight in the range of 500 to 100000; and reacting the selected cellulolytic material with the selected cellulose derivative for a time and at a temperature sufficient to produce the mixture of oligomers. The cellulose derivative is preferably selected from the group of carboxymethylcellulose, methylcellulose, methylethylcellulose, hydroxypropylmethylcellulose, hydroxypropylcellulose and mixtures thereof.

The step of selecting the cellulolytic agent may comprise selecting a hydrolytic chemical or mixture of chemicals such as a hydrolytic acid or base treatment solution (e.g. containing $H_2SO_4$, HCl, NaOH or $NH_4OH$) or an oxidative chemical or chemical solution (e.g. solutions containing oxygen, hydrogen peroxide, ozone or mixtures thereof).

The step of selecting the cellulolytic agent may also comprise selecting a microorganism which produces a cellulolytic material and preparing a cellulolytic material from a culture of the microorganism. The cellulolytic material produced by the microorganism may be purified to remove enzymes which will react with the cellulose derivative to produce mono- and disaccharides. The microorganism is preferably selected from the group of Trichoderma, Aspergillus and Penicillium. In order to prevent hydrolysis of the cellulose derivative into mono- and disaccharides the selected microorganism may alternatively be treated to alter the genes of the microorganism such that production of mono-and disaccharide generating enzymes by the genes is disenabled.

The invention further contemplates removing all or a portion, typically up to 50% by weight of a selected fat contained in a foodstuff and substituting a mixture of oligomers produced according to the invention for the removed fat; and/or, removing up to about 40% of a selected carbohydrate contained in a foodstuff and substituting a mixture of oligomers produced according to the invention for the removed carbohydrate.

The invention also contemplates separating a mixture of oligomers initially produced by a cellulolytic agent or process according to the invention into fractions of oligomers of different average molecular weight, removing up to 50% by weight of a selected fat contained in a foodstuff, and substituting one or more of the fractions for the removed fat; and/or, separating a mixture of oligomers initially produced by a cellulolytic agent or process according to the invention into fractions of oligomers of different average molecular weight, removing up to 40% by weight of a selected carbohydrate contained in a foodstuff and substituting one or more of the fractions for the removed carbohydrate.


BRIEF DESCRIPTION OF THE DRAWINGS


FIG. 1 shows molecular weight distribution patterns of a methylcellulose and its hydrolysate as described in Example 2a herein;

FIG. 2 shows molecular weight distribution patterns of hydroxypropylmethylcellulose and its hydrolysate as described in Example 2b herein;

FIG. 3 shows molecular weight distribution patterns of a carboxymethylcellulose and its hydrolysate as described in Example 2c(i) herein;

FIG. 4 shows molecular weight distribution patterns of a carboxymethylcellulose and its hydrolysate as described in Example 3 herein;

FIG. 5 shows molecular weight distribution patterns of selected fractions of the carboxymethylcellulose hydrolysate as described in Example 3 herein.

## DETAILED DESCRIPTION OF THE INVENTION

This invention relates to a soluble or suspendable mixture of oligomers derived from a cellulose derivative and its fractions. The oligomeric mixtures are characterized by having an average degree of polymerization (DP) in the range of 3-300 and a molecular weight in the range of 500-100000.

Following is a description of some exemplary embodiments of the invention wherein a cellulolytic treatment is carried out using enzymatic, chemical or physical agents/methods. Insofar as enzymatic treatments are concerned, the following description also includes a most preferred protocol for initial preparation of an enzyme.

In one embodiment of the invention a cellulose derivative may be hydrolyzed by treating the cellulose derivative with a solution of acid or base. Typical acid treatment solutions might contain sulphuric acid, hydrochloric acid, phosphoric acid, nitric acid or mixtures of two or more of the foregoing. Typical base solutions might contain a hydroxide ion containing or producing material such as an alkali hydroxide (e.g. sodium hydroxide), ammonium hydroxide, and mixtures of two or more of the foregoing. The concentration of the acid or base in the treatment solution and the treatment time and temperature may vary depending on the degree of degradation of the cellulose derivative which is desired. The person skilled in the art will recognize that higher acid or base concentrations, treatment times and treatment temperatures will generally result in a higher degree of degradation of the cellulose derivative (i.e. an oligomeric product mixture having a lower average DP and molecular weight). And, lower acid or base concentrations and treatment times and temperatures will generally produce oligomeric product mixtures of lower average DP and molecular weight. In any event where an acid or base hydrolysis treatment is utilized, the acid or base concentration and the treatment time and temperature is selected to produce a mixture of oligomers having an average DP of between 3 and 300, an average molecular weight of between 500 and 100000 and which most preferably contains less than about 25% by weight of mono- and disaccharides such as glucose and cellobiose.

In another embodiment of the invention a selected cellulose derivative may be degraded by oxidation with such agents as oxygen or hydrogen peroxide in basic solution or with ozone. Such oxidative treatments and reaction conditions therefor are well known in the art. Gaseous agents such as oxygen or ozone would typically be bubbled continuously through an aqueous solution of the cellulose derivative for a suitable time and at a suitable temperature. An oxidative treatment with peroxide might comprise treating a selected cellulose derivative with a solution of hydrogen peroxide of suitable concentration and at a suitable temperature.

The invention further contemplates oligomeric mixtures produced by physical (mechanical) depolymerization methods such as by subjecting a solution of a selected cellulose derivative to treatment with relatively high frequency sound waves with a sonicator. Other physical treatments well known in the art such as chopping or shearing a selected cellulose derivative with, for example, a high speed mixer or homogenizer may be employed to effect depolymerization.

Whatever conventional chemical (hydrolytic, oxidative or otherwise) or physical treatments are employed, the conditions and the degree of treatment are selected such that the oligomeric mixture resulting from the initial treatment has an average DP of between 3 and 300, an average molecular weight of between 500 and 100000 and contains less than about 25% by weight of mono- and disaccharides and most preferably less than about 10% by weight of mono- and disaccharides.

### Enzyme Preparation

Enzymes which may be used in some embodiments of this invention are various food-grade cellulase preparations. They can be produced from a multitude of different microorganisms such as strains of Trichoderma, Aspergillus, Penicillium, etc. A selected microorganism strain is grown by conventional means in a medium containing food grade materials such that the cellulases are produced, the microorganism is separated from the medium, the medium is collected and typically concentrated and dried. These enzymes can be used as such or in mixtures and they can be modified in many different ways known to the man skilled in the art. A most preferred enzyme preparation is produced from Trichoderma reesei, from which preparations the beta-glucosidase and/or the cellobiohydrolase activities are removed chromatographically or genetically. Beta-glucosidase and/or cellobiohydrolase activities are preferably removed from the selected cellulase preparation so as to prevent the degradation of the cellulose derivative into mono- and disaccharides. Genetic alteration of the appropriate enzyme producing microorganism may be effected with radiation or mutagenic chemical agents (or by gene inactivation by recombinant DNA methods) so as to disenable production of beta-glucosidase and cellobiohydrolase by the microorganism. Cellulase preparations suitable for use herein are, e.g., the commercially available cellulase preparations designated as the Econase series as produced by Alko Ltd., Helsinki Finland.

## Starting Materials

Preferred cellulose derivatives for use herein are carboxymethyl-, methyl-, methylethyl-, hydroxypropylmethyl- or hydroxypropylcellulose and any combinations thereof. The invention is not limited to the use of these cellulose derivatives.

## General preparation of the hydrolysate

In one embodiment of the invention, cellulose derivative hydrolysates may be prepared from soluble cellulose derivatives as defined above by an enzymatic hydrolysis utilizing a cellulase preparation having endo- 1, 4- beta-glucanase as the sole active hydrolytic agent such that only insignificant amount of mono- and disaccharides which are absorbed in human intestine (e.g., glucose) or hydrolyzed by the intestinal bacterial flora (e.g., cellobiose), are produced. On the other hand the average degree of polymerization (DP) of the oligomers formed by such a hydrolysis is lower than 300, and thus the viscosity of solutions of the hydrolysate is reduced significantly compared to the viscosity of solutions of the unhydrolysed cellulose derivatives. The specific conditions suitable for and the specific time sufficient to secure the desired hydrolysis may be readily determined for each selected cellulose derivative and each selected enzyme preparation.

Similarly in other embodiments of the invention where degradation is carried out using chemical or physical means, the average DP of the oligomers is less than 300 and the viscosity of the resulting mixture is significantly reduced. Most preferably in such embodiments, the treatment conditions are selected such that the resulting oligomeric mixtures contain less than about 5% by weight of mono- and disaccharides.

## Use of molecular weight polymers or oligomeric mixtures derived from cellulose derivatives

The degraded cellulose derivative products of the invention (and fractions thereof) dissolve rapidly in cold and hot water and are physiologically inert. Such initially formed oligomeric mixtures and selected fractions may act as thickeners, binders (e.g. in coating applications such as in the formation of conductive particle filled coatings on electrodes), stabilizers, suspending agents or flow control agents, or fillers in wide variety of applications such as in cosmetics, pharmaceuticals, plastics, paper and the like. Such products may also form films resistant to oils, greases and organic solvents and may be useful as organic resistant coatings such as coatings on clothing, paper and the like.

The cellulose derivatives used as starting materials in the present invention are as such non-caloric. Because a degradative treatment according to the present invention does not product significant amounts of metabolizable sugars, the resulting oligomeric mixtures according to this invention with their improved properties, are especially useful as low-caloric substitutes in food stuffs.

The oligomeric mixtures and fractions thereof produced according to this invention can be used for expample as new low-caloric fat sparing agents or bulking agents. These mixtures can be used to replace fat in various food stuffs, like baked goods, butter icing and custard. All or a portion of the fat can be replaced by these mixtures up to a level of at least 50%, but a level of 40% is most preferred at least when carboxymethyl hydrolysates are used. The amount which can be replaced depends on the application. The texture of the food stuff and the eating quality of the new product can thus be improved or remain unchanged.

Oligomeric mixtures and fractions thereof produced according to this invention can be used also as new low-caloric bulking agents. These mixtures can be used to replace carbohydrates such as sugar in different kinds of baked products or in other food stuffs. The amount of carbohydrate replaced with these mixtures depend on the application and average chain length of the oligomers.

By conventional means an initially degraded cellulose derivative mixture may be further separated into fractions of oligomers of differing average chain lengths. The viscosity of the various fractions will vary with the degree of average chain length of the oligomers contained within in a fraction. Depending on the particular food stuff application, the invention further contemplates selecting one or more fractions from an initial oligomeric mixture having a viscosity (average chain length) which is most appropriate for the particular food stuff application. The selection of a particular average chain length fraction and the amount of such a fraction to be used in any given food stuff application may vary according to the amount of fat or carbohydrate to be replaced, it being recognized that the higher the absolute amount of substitution agent desired to be used in a particular foodstuff, the lower the viscosity (average molecular weight) of the fraction of mixture of oligomers should be used.

The invention is described in greater detail in the following examples.

## Example 1 -- Cellulase Preparation

The beta-glucasidase activity was removed by ion exchange chromatography from the commercially available cellulase preparation, Econase CE, as so designated by Alko Ltd., Helsinki, Finland which was produced from a strain of Trichoderma reesei. The cellulase preparation (column A, Table 1) was passed through a cation exchange column (S-Sepharose FF, Pharmacia, LKB Biotechnology AB, Uppsala, Sweden) which was equilibrated with 50 mM sodium acetate pH 3.8 equilibrium buffer. The unbound protein (including oligomer producing endoglucanases) was washed out with the equilibration buffer (column B, Table 1). Beta-glucosidase activity remained bound to the column and could be separately eluted with 1M NaCl.

TABLE 1

| Enzyme | Relative Enzyme Activity (%) | |
|---|---|---|
| | A | B |
| | before ion exchange procedure | after ion exchange procedure |
| Beta-glucosidase | 100 | 0 |
| endo-1, 4, -beta-glucanase | 100 | 70 |

Endo- 1, 4- beta-glucanase and beta-glucosidase activities were measured as described by Bailey & Nevalainen (1981): Enzyme Microb. Technol. 3: 153-157. The relative enzyme activities reported in Table 1 of the Econase preparations before and after passage through an ion exchange column demonstrate the results of a typical means according to the invention of preparing an essentially beta-glucosidase free preparation for use in producing the oligomeric hydrolysates contemplated by the invention.

Although Table 1 reports relative enzyme activities, the absolute amount of enzyme used in any particular example is hereafter reported in terms of the amount of enzyme activity of the enzyme employed according to the universal activity unit of nano-katal (nkat) which stands for that amount of enzyme which produces one nanomole of reaction product in one second. (In the context of this application a hydrolysate reaction product such as an oligomer which is capable of reducing an agent such as dinitrosalicyclic acid which is reduced by the hydrolysate reaction product and subsequently measured.) The method of Bailey et. al., Enzyme Microb. Technol., Vol. 9, pp. 153-157 describes how such measurements of enzyme activity can be made using glucose as a standard.

## Example 2 -- Cellulose Derivative Hydrolyses

### a. Methylcellulose hydrolysate

30 g of methylcellulose (MC, Methocel MC, 64630, Fluka Chemie AG, CH-9470 Buchs, Switzerland) was mixed in 3 l of water and the pH of the solution was adjusted to 5.5 with 15% phosphoric acid and the temperature was raised to 40°C. 0.3 ml of the enzyme preparation having a total endo-1, 4 beta-glucanase activity of 1680 nkat from which the beta-glucosidase activity was removed chromatographically (as described in Example 1) was added to the solution. After hydrolysis for 24 hours the enzyme was inactivated by heating (90°C, 15 min.). The hydrolysate solution was subsequently cooled and freeze-dried.

The hydrolysate product contained less than 0.5% by weight of glucose and cellobiose.

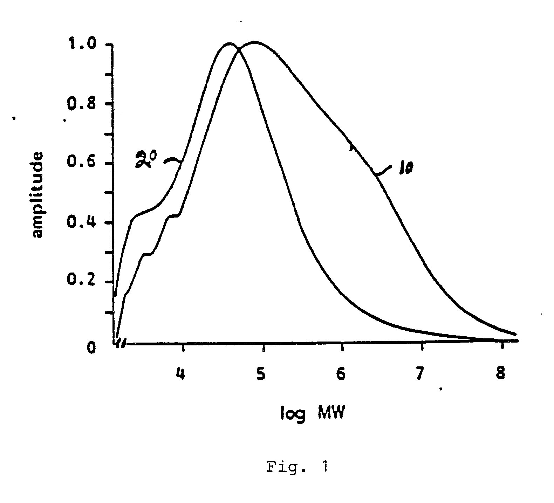

The molecular weight distribution patterns of methylcellulose, curve 10, and its hydrolysate, curve 20, are shown in FIG. 1.

The molecular weight distributions of the cellulose derivatives and their hydrolysates were determined by HPLC using a gel filtration column (TSK gel G2500PW, Toyo Soda Manufacturing Co., Ltd., Japan) with a refractive index detector (HP 1037 A) and Pharmacosmos Dextran Standards (Pharmacosmos, DK-4130, Viby Sj., Denmark). The eluent was 0.5 M sodium chloride.

b. Hydroxypropylmethylcellulose hydrolysate

20 g of hydroxypropylmethylcellulose (HPMC, H-9262, Sigma Chemical Company, St. Louis, MO, U.S.A.) was mixed in 1 l of water and the pH of the solution was adjusted to 5.5 with 15% phosphoric acid and the temperature was raised to 40° C. 0.24 ml of the enzyme preparation having a total endo-1, 4 beta-glucanase activity of 1340 nkat from which the beta-glucosidase activity was removed chromatographically (as described in Example 1) was added to the solution. After two hours another 20g of hydroxypropylmethylcellulose was added to the solution. After the hydrolysis of 22 hours the enzyme was inactivated by heating (90°C, 15 min.). Finally the hydrolysate solution was cooled and freeze-dried.

The product contained less than 0.05% by weight of glucose and cellobiose.

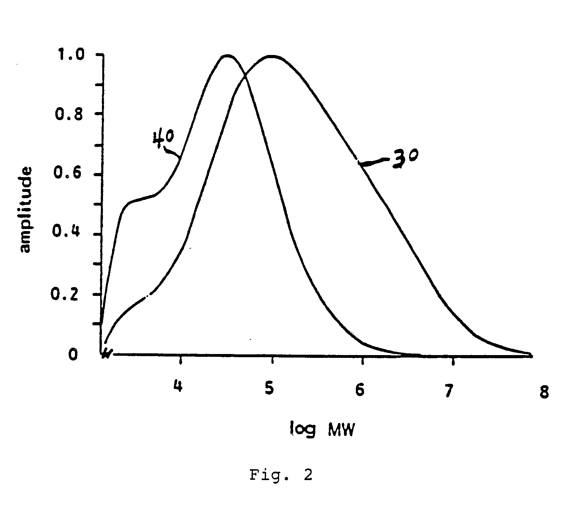

The molecular weight distribution patterns of the hydroxypropylmethylcellulose, curve 30, and its hydrolysate, curve 40, are shown in FIG. 2. The molecular weight distribution pattern was determined as described in Example 2A.

c. Carboxymethylcellulose hydrolysate

(i) Hydrolysis with Trichoderma reesei derived enzyme preparation

20 kg of carboxymethylcellulose (CMC 7MFD-type, a cellulose gum, also designated by the tradename Blanose and available from Hercules Chemical Company, 92507, Rueil-Malmaison Ceder, France; 7MFD designating a medium viscosity, food grade sodium carboxymethylcellulose having 7 out of 10 glucose units substituted with carboxymethyl) was mixed in 320 l of water and the pH of the solution was adjusted to 5.5 with 15% phosphoric acid and the temperature was raised to 40°C. 0.27 l of the enzyme preparation having a total endo-1, 4 beta-glucanase activity of 1,780,000 nkat from which the beta-glucosidase activity was removed chromatographically (as described in Example 1) was added to the CMC solution. After one hour another 20 kg of CMC was added to the solution. After hydrolysis of 23 hours the enzyme was inactivated by heating (90°C, 15 min.) Finally, the hydrolysis solution was concentrated by conventional evaporating and spray-drying.

The product contained less than 2% by weight of glucose and cellobiose. When the same hydrolysis was carried out with the original cellulase enzyme preparation of Trichoderma reesei-fungus, the amount of produced glucose and cellobiose was above 5% by weight.

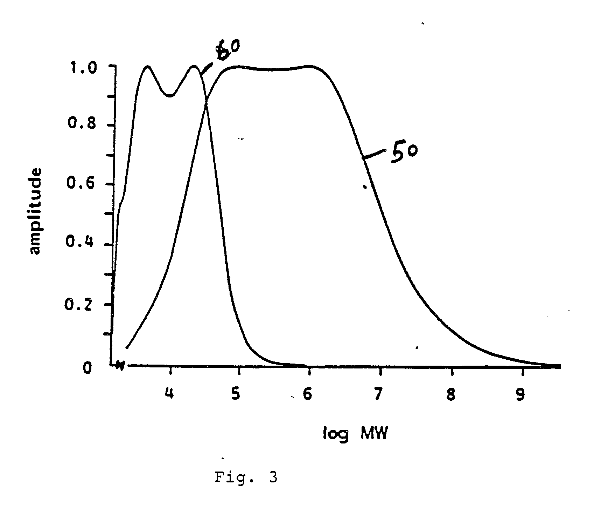

The molecular weight distribution patterns of carboxymethylcellulose, curve 50, and its hydrolysate, curve 60, are shown in FIG. 3.

The molecular weight distribution pattern was determined as described in Example 2a.

(ii) Hydrolysis with Aspergillus and Penicillium derived enzyme preparations

The enzyme preparations selected were commercially available Cellulase AP 3 (Amano Pharmaceutical Co., Ltd., Nagoya, Japan) produced using an Aspergillus strain and Cellulase CP(Sturge Enzymes, North Yorkshire, England) produced using a Penicillium strain. Carboxymethylcellulose hydrolysates were prepared as described in Example 2c(i), except that 30g of CMC-7MFD was used in 1 l of water, and the amounts of enzymes added were 0.028 g of Cellulase AP 3 (having a total endo-1, 4 beta-glucanase activity of 1350 nkat) and 0.048 g of Cellulase CP (having a total endo-1, 4 beta-glucanase activity of 1350 nkat). The viscosities and molecular weight distributions of the hydrolysates produced by either cellulase were similar (FIG. 3) to the hydrolysate produced with enzymes derived from Trichoderma reesei.

The viscosities of the various cellulose derivatives and their hydrolysates as described and prepared in Example 2 were measured using a Haake-Rotovisco viscometer with sensor systems NV (Karlsruhe, Federal Republic of Germany) (Table 2). The viscosities were measured in water solutions at 25°C. Table 2 sets forth the concentrations (by weight) of a variety of solutions all having the same viscosity.

### TABLE 2

| Concentrations of cellulose derivatives and their respective hydrolysates in solution all having a viscosity of 20 mPa.s (milli-Pascals-second) at 25°C. | |
|---|---|
| Cellulose Derivative | Concentration (by weight) |
| Methylcellulose | 2% |
| Methylcellulose hydrolysate | 5% |
| Hydroxypropylmethylcellulose | 3% |
| Hydroxypropylmethylcellulose hydrolysate | 10% |
| Carboxymethylcellulose | 2% |
| Carboxymethylcellulose hydrolysate | 20% |

As the data in Table 2 indicates, the hydrolysate of a cellulose derivative has a substantially lower viscosity than an equal amount by weight in aqueous solution of the cellulose derivative itself. Thus, the hydrolysate can be incorporated into a foodstuff in substantially higher quantity as a fat or sugar substitute than the cellulose derivative itself without compromising the texture, volume, density or the like of the foodstuff.

Example 3 -- The fractionation of carboxymethylcellulose hydrolysate

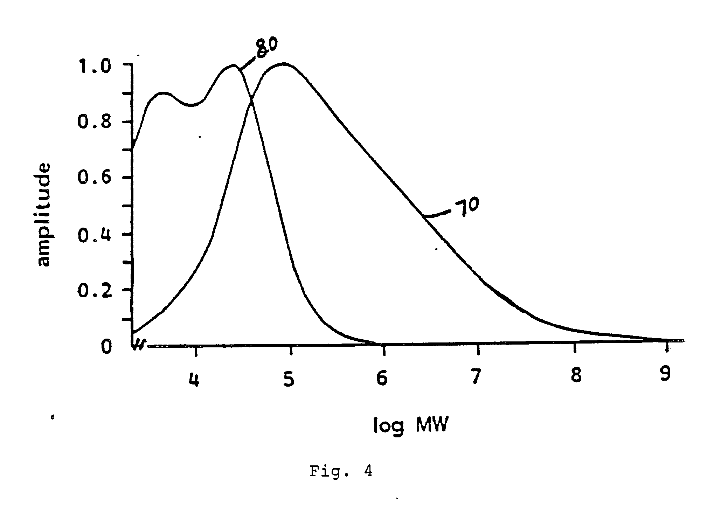

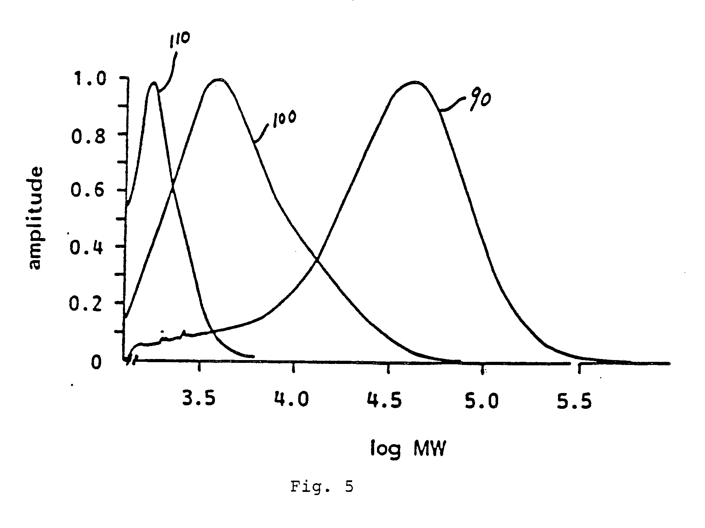

The carboxymethylcellulose hydrolysate was prepared as described in Example 2c, except that the raw material was CMC 7LFD (designating a low viscosity, food grade cellulose gum having 7 out of 10 glucose units substituted with carboxymethyl, designated under the tradename Blanose and available from Hercules Chemical Co., France) 1.6 kg CMC was used in 8 l of water and that the amount of enzyme added was 13.2 ml having a total endo- 1, 4 beta-glucanase activity of 87,000 nkat. 5 ml of the hydrolysate (0.5 g of dry matter) was further fractionated into three fractions by gel permeation chromatography (Pharmacia K 26/100 -column, Sephacryl S-200-gel, Pharmacia LKB Biotechnology AB, S-75182 Uppsala, Sweden). The eluent was distilled water, the flow rate was 14 ml/hour, and the fractionation process was carried out for 45 hours and fractions collected at intervals of 0.5 hours and pooled into three fractions (18 hours - 26 hours, curve 90, 26 hours - 32 hours, curve 100, and 32 hours - 38 hours, curve 110, FIG. 5, respectively). The molecular weight distributions of carboxymethylcellulose, curve 70, carboxymethylcellulose hydrolysate, curve 80, and the three further fractions, curves 90, 100, 110, FIGS. 4, 5 were determined by HPLC as described in Example 2.

Example 4

2 g of carboxymethylcellulose (Blanose Cellulose Gum 7 LFD, Hercules Chemical Co., 92507, Rueil-Malmaison Cedar, France) was hydrolyzed for about one hour in 100 ml of 1M sulphuric acid solution at about 100°C. After hydrolysis the solution was cooled to about room temperature, neutralized to about pH 6 with 25 ml of 25% (w/w) of NaOH solution and freeze-dried. This hydrolysis treatment produced a mixture of oligomers containing less than about 4% by weight of saccharides (glucose). The viscosity (and average DP) of this hydrolysate is similar to the viscosities (and average DP) of the hydrolysates produced by the enzymatic treatments described above utilizing enzymes derived from Trichoderma reesei.

Example 5 -- The evaluation of the fat sparing agent according to the invention in Madeira cake

Standard Madeira cakes were prepared by mixing 200 g of high ratio cake flour, 250 g of sugar-caster, 130 g of high ratio shortening, 16 g of skimmed milk powder, 3 g of salt, 12 g of baking powder, 180 g of water and 176 g of egg (defrosted). 180 g of the mix was scaled into greased tins and baked at 170°C 30 min. Madeira cakes, in which 40% of the fat was substituted (i.e., 130 g of shortening was reduced by 40% to 78 g) with 1) carboxymethylcellulose hydrolysate (fat sparing agent according to the invention), 2) carboxymethylcellulose or 3) potato maltodextrin (a conventional fat sparing agent) were compared to the standard Madeira cakes and to each other.

According to a trained panel the results were as described in Table 3.

## TABLE 3

| Product tested | Level of Fat Substitution (%) | Fat Sparing Agent Concentration in the Product Tested (% by weight) | Summary of Performance |
|---|---|---|---|
| Standard | -- | -- | A good volume, open-textured cake was produced. It gave a light eat with a 'melt-in-the-mouth' eating quality. |
| Same as Standard with Carboxymethylcellulose hydrolysate used as fat substitute | 40.0 | 2.2 | A good volumed, open-textured cake was produced. It gave an eating quality close to that of standard, but not quite as good. |
| Same as Standard with Carboxymethylcellulose used as fat substitute | 40.0 | 0.3 | A lower-volumed, denser cake was produced. The eating quality was very poor -- a pronounced gumminess was present and there was no 'melt-in-the-mouth' characteristic. |

| Same as Standard with Potato maltodextrin used as fat substitute | 40.0 | 0.3 | A good volumed, open-textured cake was produced. The eating quality was close to that of standard and carboxy-methyl-cellulose hydrolysate. Again it was slightly poorer than the standard. |
|---|---|---|---|

Example 6 -- Evaluation of the fat sparing agent according to the invention in butter icing

A standard butter icing was prepared by mixing 179 g of butter (unsalted), 225 g of icing sugar and 96 g of water. The butter icings, in which 30% of fat was substituted (i.e., 179 g of butter was reduced by 30% to 125.3 g) with 1) carboxymethylcellulose hydrolysate (fat sparing agent according to this invention), 2) carboxymethylcellulose or 3) potato maltodextrin and all were compared to the standard butter icing and to each other.

According to a trained panel the results were as described in Table 4.

Example 7 -- Evaluation of the bulking agent according to the invention in marzipan

A standard marzipan product was prepared by mixing 21.8 g of icing sugar, 21.8 g of caster sugar, 43.7 g of ground almonds, 0.8 g of vanilla flavoring, 10.9 g of eggs (lightly beaten) and 1 g of lemon juice for every 100 g of product. The mixture was formed into a ball, kneaded lightly, rolled out and cut out. Marzipan, in which 10% of sugar was substituted with carboxymethylcellulose hydrolysate (bulking agent according to the invention), had good almond flavor and same color as the standard marzipan product. It was also easy to roll and cut out. Higher percentage sugar replacement may be required to reduce calories significantly. Appropriate lower viscosity DP-fractions of carboxymethylcellulose hydrolysate may be employed in greater than 10% by weight amounts, preferably up to as much as about 40% while still maintaining normal texture.

TABLE 4

| Product tested | Level of Fat Substitution (%) | Fat Sparing Agent Concentration in the Product Tested (% by weight) | Summary of Performance |
|---|---|---|---|
| Standard | -- | -- | An acceptable icing was produced. It was firm, smooth textured and held onto water extremely well. It was much better than the icing made with carboxymethylcellulose and potato maltodextrin. |
| Same as Standard with Carboxymethylcellulose hydrolysate used as fat substitute | 30.0 | 4.3 | A very acceptable icing was produced. It was firm, smooth textured and held onto the water extremely well. It was better than the standard icing. |
| Same as Standard with ·Carboxymethylcellulose used as fat substitute | 30.0 | 0.5 | A very poor, unacceptable icing was produced. The system did not hold onto the water at all well. |
| Same as Standard with Potato maltodextrin used as fat substitute | 30.0 | 2.7 | Again a poor, unacceptable icing was produced. The water was not held within the system. |

## Claims

1. A water-soluble degradation product of a cellulose derivative comprising a cellulose derivative degraded to form a mixture of oligomers having an average degree of polymerization in the range of 3 to 300 and a molecular weight of 500 to 100000.

2. A product according to claim 1 wherein the soluble cellulose derivative is carboxymethylcellulose, methylcellulose, methylethylcellulose, hydroxypropylmethylcellulose, hydroxypropylcellulose or a mixture thereof.

3. A product according to claim 1 or 2 wherein the cellulose derivative is hydrolysed to produce the mixture by treating the cellulose derivative with a solution of acid or base.

4. A product according to claim 3 wherein the concentration of the acid or the base in the solution is such as to hydrolyse the cellulose derivative into a mixture of hydrolysis products containing less than about 25% by weight of monosaccharides, disaccharides or mixtures thereof.

5. A product according to claim 1 wherein the cellulose derivative is oxidized to produce the mixture by treating the cellulose derivative with oxygen, peroxide, ozone or a compbination thereof.

6. A product according to claim 5 wherein the time, temperature and degree of treatment are such as to oxidize the cellulose derivative into a mixture of oxidation products containing less than about 25% by weight of monosaccharides, disaccharides or mixtures thereof.

7. A product according to claim 1 or 2 wherein the cellulose derivative is physically cleaved to produce the mixture by subjecting the cellulose derivative to physical chopping, physical shearing, sonication or a combination thereof.

8. A product according to claim 7 wherein the cellulose derivative is physically treated to produce a mixture of cleaved products containing less than about 25% by weight of monosaccharides, disaccharides or mixtures thereof.

9. A method for producing a mixture of oligomers from a cellulose derivative which comprises degrading the cellulose derivative into a mixture of oligomers having an average degree of polymerization in the range of 3 to 300 and a molecular weight in the range of 500 to 100000.

10. A method according to claim 9 wherein the cellulose derivative is carboxymethylcellulose, methylcellulose, methylethylcellulose, hydroxypropylcellulose, hydroxypropylmethylcellulose or a mixture thereof.

11. A method according to claim 9 or 10 wherein the step of degrading comprises treating the cellulose derivative with an aqueous solution of acid or base.

12. A method according to claim 11 wherein the concentration of the acid or base solution and the treatment time and temperature are such as to hydrolyse the cellulose derivative into a mixture of hydrolysis products containing less than about 25% by weight of monosaccharides, disaccharides or mixtures thereof.

13. A method according to claim 9 or 10 wherein the step of degrading comprises treating the cellulose derivative with an oxidizing agent in aqueous solution.

14. A method according to claim 13 wherein the oxidizing agent is oxygen, peroxide, ozone or a combination thereof.

15. A method according to claim 13 or 14 wherein the treatment time and temperature are such as to oxidize the cellulose derivative into a mixture of oxidation products containing less than about 25% by weight of monosaccharide, disaccharides or mixtures thereof.

16. A method according to claim 9 or 10 wherein the step of degrading comprises physically cleaving the cellulose derivative into the mixture of oligomers.

17. A method according to claim 16 wherein the cellulose derivative is cleaved by one or more of chopping, shearing and sonication.

18. A method according to claim 16 or 17 wherein the cellulose derivative is physically cleaved for a time and to a degree selected to produce a mixture of cleaved products containing less than about 25% by weight of monosaccharides, disaccharides or mixtures thereof.

Fig. 1

Fig. 2

Fig. 3

Fig. 4

Fig. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 101, no. 8, 20th August 1984, page 81, abstract no. 56734p, Columbus, Ohio, US; & JP-A-59 41 301 (DAICEL CHEMICAL INDUSTRIES, LTD) 07-03-1984 * Abstract * | 1,3,9, 11 | C 08 B 15/02 C 08 B 11/20 |
| X | DE-A-2 016 203 (DOW) * Table 1; examples * | 1,2,5,9 ,13,14 | |
| A | CH-A- 423 225 (RAYONIER) * Claim * | 1,5,9, 13,14 | |
| A | GB-A-1 122 006 (SHIN-ETSU) * Page 1, column 1; claims * | 1-18 | |
| A | TAPPI (TECHNICAL ASSOCIATION OF THE PULP AND PAPER INDUSTRY), vol. 61, no. 5, May 1978, pages 101-105, Atlanta, US; H. STEEGE et al.: "Microcrystalline cellulose powders" * Page 102, column 1; page 103, column 3 * | | TECHNICAL FIELDS SEARCHED (Int. Cl.5) C 08 B |

The present search report has been drawn up for all claims

| Place of search | Date of completion of the search | Examiner |
|---|---|---|
| THE HAGUE | 16-05-1990 | SOMERVILLE F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)